# DEMANDE DE BREVET EUROPEEN

(11) **EP 1 433 474 A1**
(43) Date de publication de la demande: **30.06.2004**
(21) Numéro de dépôt: 03293292.3
(22) Date de dépôt: 23.12.2003
(51) Int. Cl.: A61K 7/13

(54) **Composition tinctoriale contenant un colorant direct polycationique dissymétrique particulier, procédé de teinture, utilisation et dispositifs à plusieurs compartiments**

(30) Priorité: 23.12.2002 FR 0216566
(71) Demandeur: L'OREAL, 75008 Paris (FR)
(72) Inventeur: Lagrange, Alain, 77700 Coupvray (FR)
(74) Mandataire: Dossmann, Gérard

(57) **Abrégé**

L'invention a pour objet une composition tinctoriale pour la teinture des fibres kératiniques humaines et plus particulièrement des cheveux, comprenant un colorant direct polycationique dissymétrique de formule (I),

Col₁-Z-Col₂ (I)

dans laquelle
Col₁ et Col₂ sont des colorants différents de par leur structure choisis dans le groupe formé par les colorants azoïques, les colorants méthiniques, les colorants azométhiniques, les colorants phénothiaziniques, les colorants triarylméthaniques, les colorants xanthéniques, les colorants phénanthridiniques, les colorants phtalocyanines,
Z est un groupement hydrocarboné en C₁-C₂₀, linéaire ou ramifié, saturé, insaturé ou cyclique comprenant au moins un atome d'azote et porteur d'au moins une charge cationique,
ainsi que les procédés de teinture des fibres kératiniques humaines la mettant en oeuvre, l'utilisation des colorants de formule (I) à titre de colorants directs, ainsi que des dispositifs à plusieurs compartiments.

## Description

L'invention a pour objet une composition tinctoriale pour la teinture des fibres kératiniques humaines et plus particulièrement des cheveux, comprenant un colorant polycationique direct dissymétrique particulier, ainsi que les procédés de teinture des fibres kératiniques humaines mettant en oeuvre une telle composition.

Il est connu de teindre les fibres kératiniques humaines et en particulier les cheveux avec des compositions tinctoriales contenant des précurseurs de colorant d'oxydation, appelés généralement bases d'oxydation. Ces bases d'oxydation sont des composés incolores ou faiblement colorés qui, associés à des produits oxydants, donnent naissance par un processus de condensation oxydative à des composés colorés.

On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Ce procédé de coloration d'oxydation consiste à appliquer sur les fibres kératiniques, des bases d'oxydation ou un mélange de bases d'oxydation et de coupleurs avec un agent oxydant, par exemple de l'eau oxygénée, à laisser poser, puis à rincer les fibres. Les colorations qui en résultent sont permanentes, puissantes, et résistantes aux agents extérieurs, notamment à la lumière, aux intempéries, aux lavages, à la transpiration et aux frottements. Généralement appliquées à pH basique, il permet d'obtenir une teinture et simultanément un éclaircissement de la fibre qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris, et dans le cas de cheveux naturellement pigmentés, de faire ressortir la couleur, c'est-à-dire de la rendre plus visible.

Il est aussi connu de teindre les fibres kératiniques humaines par une coloration directe. Le procédé classiquement utilisé en coloration directe consiste à appliquer sur les fibres kératiniques des colorants directs qui sont des molécules colorées et colorantes ayant une affinité pour les fibres, à laisser poser, puis à rincer les fibres.

Il est connu par exemple d'utiliser des colorants directs nitrés benzèniques, anthraquinoniques, nitropyridiniques, des colorants azoïques, xanthéniques, acridiniques aziniques ou des colorants triarylméthaniques.

Les colorations qui en résultent sont des colorations particulièrement chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux. En outre, leur sensibilité à la lumière est dépendante de leur répartition uniforme ou en agrégats dans la fibre kératinique.

Il est connu d'utiliser des colorants directs en combinaison avec des agents oxydants. Cependant, les colorants directs sont généralement sensibles à l'action des agents oxydants tels que l'eau oxygénée, et les agents réducteurs tels que le bisulfite de sodium, ce qui les rend généralement difficilement utilisables dans les compositions de teinture directe éclaircissante à base d'eau oxygénée et à base d'un agent alcalinisant ou dans des compositions de teinture d'oxydation en association avec des précurseurs du type bases d'oxydation ou coupleurs.

Par exemple, il a été proposé dans les demandes de brevets FR-1 584 965 et JP-062 711 435 de teindre les cheveux avec des compositions de teinture à base de colorants directs nitrés et/ou de colorants dispersés azoïques et d'eau oxygénée ammoniacale en appliquant sur les cheveux un mélange desdits colorants et dudit oxydant, réalisé juste avant l'emploi. Mais les colorations obtenues se sont révélées insuffisamment tenaces et disparaissent aux shampooings en laissant apparaître l'éclaircissement de la fibre capillaire. Une telle coloration devient inesthétique en évoluant au cours du temps.

On a également proposé dans les demandes de brevets JP-53 95693 et JP-55 022638 de teindre les cheveux avec des compositions à base de colorants directs cationiques de type oxazine et d'eau oxygénée ammoniacale, en appliquant sur les cheveux, dans une première étape, de l'eau oxygénée ammoniacale, puis dans une seconde étape, une composition à base du colorant direct oxazinique. Cette coloration n'est pas satisfaisante, en raison du fait qu'elle nécessite un procédé rendu trop lent par les temps de pose des deux étapes successives. Si par ailleurs on applique sur les cheveux un mélange extemporané du colorant direct oxazinique avec de l'eau oxygénée ammoniacale, on ne colore pas, ou du moins, on obtient une coloration de la fibre capillaire qui est presque inexistante.

Plus récemment, la demande de brevet FR 2 741 798 a décrit des compositions tinctoriales contenant des colorants directs comportant au moins un atome d'azote quaternisé du type azoïque ou azométhine, lesdites compositions étant à mélanger extemporanément à pH basique à une composition oxydante. Ces compositions permettent d'obtenir des colorations avec des reflets homogènes, tenaces et brillants. Cependant, elles ne permettent pas de teindre les fibres kératiniques avec autant de puissance qu'avec des compositions de coloration d'oxydation.

Il existe donc un réel besoin de rechercher des colorants directs chromatiques qui permettent de teindre les fibres kératiniques humaines aussi puissamment que les colorants d'oxydation, qui soient aussi stables qu'eux à la lumière, soient également résistants aux intempéries, aux lavages et à la transpiration, et en outre, suffisamment stables en présence d'agents oxydants et réducteurs pour pouvoir obtenir simultanément un éclaircissement de la fibre soit par utilisation de compositions directes éclaircissantes les contenant, soit par l'utilisation de compositions de coloration d'oxydation à base de précurseurs de colorants d'oxydation les contenant. Il existe aussi un réel besoin de rechercher des colorants directs qui permettent d'obtenir des montées de couleur comparables à celles obtenus avec les précurseurs de colorants d'oxydation.

En outre, la demanderesse a cherché à obtenir des colorants présentant avantageusement une bonne innocuité, ne dégradant pas la fibre kératinique et ayant une sélectivité diminuée par rapport aux colorants classiques.

Ces buts sont atteints avec la présente invention qui a pour objet une composition pour la teinture des fibres kératiniques humaines et plus particulièrement des cheveux, comprenant au moins un colorant direct polycationique dissymétrique de formule (I) suivante :

Col₁-Z-Col₂ (I)

dans laquelle
Col₁ et Col₂ sont des colorants différents de par leur structure. Selon cette structure, ils peuvent posséder ou non le même nombre de charges cationiques. Ils sont choisis dans le groupe formé par les colorants azoïques, les colorants méthiniques, les colorants azométhiniques, les colorants phénothiaziniques, les colorants triarylméthaniques, les colorants xanthéniques, les colorants phénanthridiniques et les colorants phtalocyanines,
Z est un groupement hydrocarboné en C₁-C₂₀, linéaire ou ramifié, saturé, insaturé ou cyclique comprenant au moins un atome d'azote et porteur d'au moins une charge cationique. Les compositions selon l'invention présente en outre l'avantage de permettre d'obtenir des couleurs naturelles.
Au sens de la présente invention on entend :
- par colorant azoïque, une molécule ou un reste de molécule absorbant les radiations lumineuses dans le domaine visible (400-750 nm) et comportant dans sa structure au moins un enchaînement (A) non inclus dans un cycle

   -N=N- (A)
- par colorant méthinique, une molécule ou un reste de molécule absorbant les radiations lumineuses dans le domaine visible (400-750 nm) et comportant dans sa structure au moins un enchaînement (B) non inclus dans un cycle

   -C=C- (B)
- par colorant azométhinique, une molécule ou un reste de molécule absorbant les radiations lumineuses dans le domaine visible (400-750 nm) et comportant dans sa structure au moins un enchaînement (C) non inclus dans un cycle

   -N=C- (C)
- par colorant triarylméthanique, une molécule ou un reste de molécule absorbant les radiations lumineuses dans le domaine visible (400-750 nm) et comportant dans sa structure au moins un enchaînement (D) 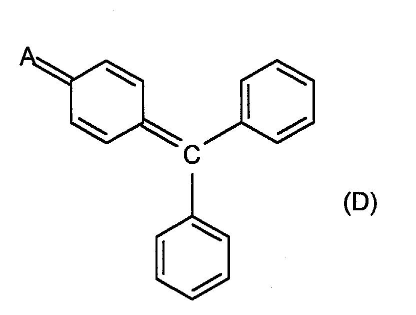 A désignant un atome d'oxygène ou d'azote
- par colorant xanthènique, une molécule ou un reste de molécule absorbant les radiations lumineuses dans le domaine visible (400-750 nm) et comportant dans sa structure au moins un enchaînement (E) 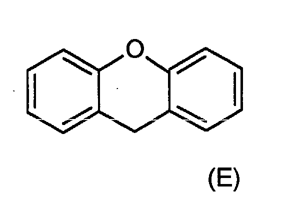
- par colorant phénanthridinique, une molécule ou un reste de molécule absorbant les radiations lumineuses dans le domaine visible (400-750 nm) et comportant dans sa structure au moins un enchaînement (F) 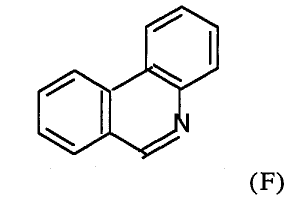
- par colorant phtalocyanine, une molécule ou un reste de molécule absorbant les radiations lumineuses dans le domaine visible (400-750 nm) et comportant dans sa structure au moins un enchaînement (G) 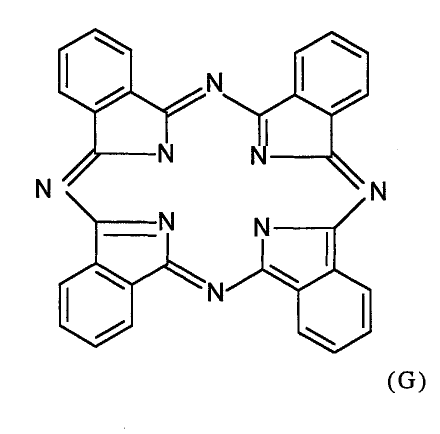
- par colorant phénotiazinique, une molécule ou un reste de molécule absorbant les radiations lumineuses dans le domaine visible (400-750 nm) et comportant dans sa structure au moins un enchaînement (H)

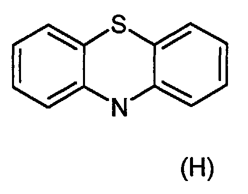

Au sens de la présente invention, on entend par "charge cationique", tout atome d'azote quaternisé.

Au sens de la présente demande, on entend par "groupement hydrocarboné en C₁-C₂₀", une chaîne aliphatique en C₁-C₂₀, de préférence en C₆-C₁₈ pouvant être interrompue par un ou plusieurs (de 1 à 5) cycles hétéroatomes comme l'azote, l'oxygène, le soufre ou le phosphore, cette chaîne pouvant comprendre un ou plusieurs (de 1 à 5) cycles aromatiques, un ou plusieurs (de 1 à 5) hétérocycles aromatiques ou saturés, un ou plusieurs (de 1 à 5) cycles aliphatiques ou pouvant être substitués par un ou plusieurs (de 1 à 5) groupements choisis parmi les groupements hydroxy, carboxy, alcoxy( en C₁-C₄)carbonyles, hydrogénocarbonyles, alcoxy en C₁-C₄, acyle en C₂-C₄, amino, mono ou dialkylamino, mono ou di(hydroxyalkyl en C₁-C₄)amino, cyano, nitro, sulfonato.

Ladite chaîne peut comporter de préférence une ou plusieurs liaisons doubles et/ou une ou plusieurs liaisons triples. Cette chaîne hydrocarbonée pourra également comprendre des groupements aromatiques tels qu'un cycle benzènique ou naphtalènique. La chaîne peut aussi former un ou plusieurs cycles carbonés comportant de 3 à 6 chaînons.

Le rattachement des colorants Col 1 ou Col 2 au groupe Z peut se faire directement sur les groupements azotés cationiques des colorants sur un ou plusieurs autres atomes de la molécule colorante ou via un ou plusieurs bras de liaison.

Selon la présente invention, la structure du groupement Z peut ainsi répondre à la formule (II) :

- Z₁-Z₂-Z₃- (II)

dans laquelle :
Z₁ et Z₃, indépendamment l'un de l'autre, représentent un groupe hétérocyclique, le ou les hétéroatomes du groupe sont choisis parmi l'azote, l'oxygène, le soufre ou le phosphore. Ces groupes hétérocycliques peuvent comporter de 5 à 8 chaînons et éventuellement être condensés avec un noyau benzénique. Ils peuvent être substitués par un ou plusieurs groupements alkyle en C₁-C₄, alcoxy en C₁-C₄, hydroxy, amino, mono ou dialkylamino, mono ou di(hydroxyalkyle C₁-C₄)amino.
Z₂ est un groupe hydrocarboné linéaire ou ramifié comprenant de 0 à 10, de préférence de 2 à 6 atomes de carbone.

De préférence, les groupes hétérocycliques sont choisis dans le groupe formé par les groupements pyrroles, imidazoles, isoimidazoles, pyridines, pyrazoles.

Selon la présente invention, la structure du groupement Z peut aussi répondre à la formule (III) : 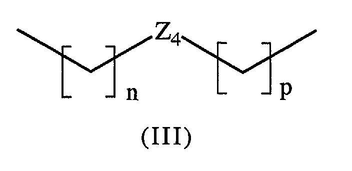 dans laquelle :
n représente un nombre entier de 1 à 10, de préférence de 2 à 5,
p représente un nombre entier de 1 à 10, de préférence de 2 à 5,
Z₄ représente un groupe cationique et de préférence dicationique comprenant entre 2 et 16 atomes de carbone, de préférence entre 5 et 12 atomes de carbone, ce groupe étant aliphatique, saturé ou insaturé, carbocyclique ou polycarbocyclique, aromatique ou polyaromatique, hétéroaromatique ou polyhétéroaromatique, substitué ou non substitué par un à cinq substituants choisis parmi les groupements hydroxy, carboxy, alcoxy en C₁-C₄carbonyles, hydrogénocarbonyles, alcoxy en C₁-C₄, amino, mono ou dialkylamino, mono ou di(hydroxyalkyl en C₁-C₄)amino, cyano, nitro, sulfonato.

Un cas particulier de groupement Z de formule (III) est constitué par les groupements Z de formule (IV).

Z peut être un groupement aliphatique, et plus préférentiellement, un groupe de formule (IV) : 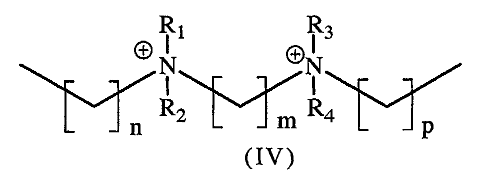 dans laquelle
n représente un nombre entier de 1 à 10, de préférence de 2 à 5,
m représente un nombre entier de 1 à 15, de préférence de 2 à 10
p représente un nombre entier de 1 à 10, de préférence de 2 à 5,
R₁, R₂, R₃, R₄ désignent indépendamment les uns des autres un radical alkyle en C₁-C₆.

De préférence, les groupements Col₁ et Col₂ désignent indépendamment l'un de l'autre, un colorant azoïque ou un colorant méthinique.

Les composés de l'invention sont des composés connus en eux-mêmes.

Parmi ceux-ci, on peut citer les composés suivantes :
Ammonium, [2-[p-[[2-chloro-4-(méthylsulfonyl)phényl]azo]-N-éthylanilino]éthyl][2-[N-éthyl-p-[(5-nitro-2-thiazolyl)azo]anilino]éthyl]diméthyl-, p-toluènesulfonate
(2-{[4-(2-Chloro-4-nitro-phénylazo)-2-methoxy-5-méthyl-phényl]-méthyl-amino}-éthyl)-(2-{[4-(2-chloro-4-nitro-phénylazo)-phényl]-éthyl-amino}-éthyl)-diméthyl-ammonium
Tétraiodure de Quinolinium, 1-[3-[[3-[diméthyl[3-[2-[(3-méthylbenzothiazolium-2-yl)méthylène]-3(2H)-benzothiazolyl]propyl]ammonio]propyl]diméthylammonio]propyl]-4-[(3-méthyl-2(3H)-benzothiazolylidène)méthyl]-
Tétraiodure de Quinolinium, 1-[3-[[3-[diméthyl[3-[4-[(3-méthyl-2(3H)-benzothiazolylidène)méthyl]quinolinio]propyl]ammonio]propyl]diméth ylammonio]propyl]-4-[5-(3-méthyl-2(3H)-benzothiazolylidène)-1,3-pentadiényl]-
Tétraiodure de Quinolinium, 1-[3-[[3-[diméthyl[3-[2-[5-(3-méthyl-2(3H)- benzothiazolylidène)-1,3-pentadiényl]benzothiazolium-3-yl]propyl]ammonio]propyl]diméthylammonio]propyl]-4-[(3-méthyl-2(3H)- benzothiazolylidène)méthyl]-
Tétraiodure de Quinolinium, 1-[5-[diméthyl[3-[diméthyl[3-[4-[(3-méthyl-2(3H)- benzothiazolylidène)méthyl]quinolinio]propyl] ammonio]propyl]ammonio]pentyl]-4-[3-(3-méthyl-2(3H)-benzothiazolylidène)-1-propenyl]-
Tétraiodure de Quinolinium, 1-[3-[[3-[[4-[2-[5-(1,3-dihydro-1,3,3-triméthyl-2H-indol-2-ylidène)-1,3-pentadiényl]benzothiazolium-3-yl]butyl]diméthylammonio]propyl]diméthylammonio]propyl]-4-[(3-méthyl-2(3H)-thiazolylidène)méthyl]-
Tétraiodure de Quinolinium, 1-[3-[[3-[[3-[2-[5-(1,3-dihydro-1,3,3-triméthyl-2H-indol-2-ylidène)-1,3-pentadiényl]benzothiazolium-3-yl]propyl]diméthylammonio]propyl]diméthylammonio]propyl]-4-[(3-méthyl-2(3H)-thiazolylidène)méthyl]-
Tétrachlorure de Quinolinium, 1-[3-[[3-[diméthyl[3-[4-[(3-méthyl-2(3H)- benzothiazolylidène)méthyl]quinolinio]propyl] ammonio]propyl]diméthylammonio]propyl]-4-[5-(3-méthyl-2(3H)-benzothiazolylidène)-1,3-pentadiényl]-
Tétrachlorure de Quinolinium, 1-[3-[[3-[diméthyl[3-[2-[5-(3-méthyl2(3H)- benzothiazolylidène)-1,3-pentadiényl]benzothiazolium-3-yl]propyl]ammonio]propyl]diméthylammonio]propyl]-4-[(3-méthyl-2(3H)- benzothiazolylidène)méthyl]-
Tétrachlorure de Quinolinium, 1-[4-[[3-[diméthyl[3-[4-[(3-méthyl-2(3H)- benzothiazolylidène)méthyl]quinolinio]propyl] ammonio]propyl]diméthylammonio]butyl]-4-[3-(3-méthyl-2(3H)-benzothiazolylidène)-1-propenyl]-
Tétraiodure de Quinolinium, 1-[3-[[3-[[5-[2-[5-(1,3-dihydro-1,3,3-triméthyl-2H-indol-2-ylidène)-1,3-pentadiényl]benzothiazolium-3-yl]pentyl]diméthylammonio]propyl]diméthylammonio]propyl]-4-[(3-méthyl-2(3H)-benzothiazolylidène)méthyl]-,
Tétrachlorure de Quinolinium, 1-[3-[[3-[[4-[2-[5-(1,3-dihydro-1,3,3-triméthyl-2H-indol-2-ylidène)-1,3-pentadiényl]benzothiazolium-3-yl]butyl]diméthylammonio]propyl]diméthylammonio]propyl]-4-[(3-méthyl-2(3H)-benzothiazolylidène)méthyl]-
Tétrachlorure de Quinolinium, 1-[3-[[3-[[3-[2-[5-(1,3-dihydro-1,3,3-triméthyl-2H-indol-2-ylidène)-1,3-pentadiényl]benzothiazolium-3-yl]propyl]diméthylammonio]propyl]diméthylammonio]propyl]-4-[(3-méthyl-2(3H)-benzothiazolylidène)méthyl]-
Tétrachlorure de Quinolinium, 1-[6-[[3-[diméthyl[3-[4-[(3-méthyl-2(3H)- benzothiazolylidène)méthyl]quinolinio]propyl] ammonio]propyl]diméthylammonio]hexyl]-4-[3-(3-méthyl-2(3H)-benzothiazolylidène)-1-propenyl]-
Tétrachlorure de Quinolinium, 1-[5-[[3-[diméthyl[3-[4-[(3-méthyl-2(3H)- benzothiazolylidène)méthyl]quinolinio]propyl]ammonio] propyl]diméthylammonio]pentyl]-4-[3-(3-méthyl-2(3H)-benzothiazolylidène)-1-propenyl]-,
Tétraiodure de Naphtho[1,2-d]thiazolium, 2,2'-[1,3-propanediylbis[(diméthyliminio)-3,1-propanediyl-1(4H)-quinolinyl-4-ylidèneméthylidyne]]bis[1-méthyl-
Tétrachlorure de Phénanthridinium, 3,8-diamino-5-[3-[[3-[diméthyl[3-[4-[(3-méthyl-2(3H)- benzothiazolylidène)méthyl]quinolinio]propyl] ammonio]propyl]diméthylammonio]propyl]-6-phényl]-
Quinolinium, 1-[3-[diméthyl[3-[diméthyl[3-[4-[(3-méthyl-2(3H)-benzothiazolylidène)méthyl]quinolinio]propyl]iminio]propyl]iminio] propyl]- 4-[3-(3-méthyl-2(3H)-benzothiazolylidène)-1-propenyl]-

La concentration en colorant(s) direct(s) polycationique de formule (I) peut varier d'environ 0,001% à 5% en poids par rapport au poids total de la composition tinctotriale, et de préférence d'environ 0,05% à 2%.

De préférence, les compositions de l'invention contiennent au moins un adjuvant cosmétique choisi parmi les monoalcools tels que les alcanols, les polyols, les agents tensio-actifs anioniques, cationiques, non ioniques, amphotères ou zwittérioniques, ou leurs mélanges, les agents épaississants minéraux ou organiques, et en particulier les polymères associatifs anioniques, cationiques, non ioniques et amphotères.

Les agents épaississant sont notamment choisis dans le groupe constitué par :
(i) les épaississants associatifs;
(ii) les homopolymères d'acide acrylique réticulés ;
(iii) les copolymères réticulés d'acide (méth)acrylique et d'acrylate d'alkyle(C₁-C₆)
(iv) les homopolymères et copolymères non-ioniques contenant des monomères à insaturation éthylénique de type ester et/ou amide ;
(v) les homopolymères d'acrylate d'ammonium ou les copolymères d'acrylate d'ammonium et d'acrylamide ;
(vi) les polysaccharides
vii) les alcools gras en C₁₂-C₃₀

Par l'expression "épaississant associatif", on entend selon l'invention, un épaississant amphiphile comportant à la fois des motifs hydrophiles et des motifs hydrophobes en particulier comportant au moins une chaîne grasse en C8-C30 et au moins un motif hydrophile.

On peut utiliser comme épaississants associatifs selon l'invention les polymères associatifs choisis parmi :
(i) les polymères amphiphiles non-ioniques comportant au moins une chaîne grasse et au moins un motif hydrophile ;
(ii) les polymères amphiphiles anioniques comportant au moins un motif hydrophile, et au moins un motif à chaîne grasse ;
(iii) les polymères amphiphiles cationiques comportant au moins un motif hydrophile, et au moins un motif à chaîne grasse ;
(iv) les polymères amphiphiles amphotères comportant au moins un motif hydrophile, et au moins un motif à chaîne grasse ;
les chaînes grasses ayant de 10 à 30 atomes de carbone.

Les polymères amphiphiles non-ioniques comportant au moins une chaîne grasse et au moins un motif hydrophile sont choisis de préférence parmi :
(1) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
(2) les hydroxypropylguars modifiés par des groupements comportant au moins une chaîne grasse tel que le produit ESAFLOR HM 22 (chaîne alkyle en C₂₂) vendu par la société LAMBERTI, les produits MIRACARE XC95-3 (chaîne alkyle en C₁₄) et RE205-1 (chaîne alkyle en C₂₀) vendus par la société RHODIA CHIMIE.
(3) les uréthanes polyéthers comportant au moins une chaîne grasse telle que des groupes alkyle ou alcényle en C₁₀-C₃₀, comme les produits DAPRAL T 210 et DAPRAL T 212 vendus par la société AKZO ou les produits ACULYN 44 et ACULYN 46 commercialisés par la société ROHM&HAAS.
(4) les copolymères de vinyl pyrrolidone et de monomères hydrophobes à chaîne grasse ;
   on peut citer à titre d'exemple :
   - les produits ANTARON V216 ou GANEX V216 (copolymère vinylpyrrolidone / hexadécène) vendu par la société I.S.P.
   - les produits ANTARON V220 ou GANEX V220 (copolymère vinylpyrrolidone / eicosène) vendu par la société I.S.P.
(5) les copolymères de méthacrylates ou d'acrylates d'alkyles en C₁-C₆ et de monomères amphiphiles comportant au moins une chaîne grasse tels que par exemple le copolymère méthacrylate de méthyle/acrylate de stéaryle oxyéthyléné vendu par la société GOLDSCHMIDT sous la dénomination ANTIL 208.
(6) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

Parmi les polymères amphiphiles anioniques selon l'invention comportant au moins un motif hydrophile, et au moins un motif à chaîne grasse, on préfère ceux comportant au moins un motif éther d'allyl à chaîne grasse et au moins un motif hydrophile constitué par un monomère anionique insaturé éthylénique, plus particulièrement par un acide carboxylique vinylique et tout particulièrement par un acide acrylique, un acide méthacrylique ou leurs mélanges, le motif éther d'allyl à chaîne grasse correspondant au monomère de formule (V) suivante :

CH₂ = C(R1) CH₂ O Bₙ R (V)

dans laquelle R1 désigne H ou CH₃, B désigne le radical éthylèneoxy, n est nul ou désigne un entier allant de 1 à 100, R désigne un radical hydrocarboné choisi parmi les radicaux alkyl, arylalkyl, aryl, alkylaryl, cycloalkyl, comprenant de 10 à 30 atomes de carbone, de préférence 10 à 24, et plus particulièrement encore de 12 à 18 atomes de carbone.
Un motif de formule (V) plus particulièrement préféré selon la présente invention est un motif dans lequel R1 désigne H, n est égal à 10, et R désigne un radical stéaryl (C₁₈).

Des polymères amphiphiles anioniques de ce type sont décrits et préparés, selon un procédé de polymérisation en émulsion, dans le brevet EP-0216479 B2.

Parmi ces polymères amphiphiles anioniques, on préfère particulièrement selon l'invention, les polymères formés à partir de 20 à 60% en poids d'acide acrylique et/ou d'acide méthacrylique, de 5 à 60% en poids de (méth)acrylates d'alkyls inférieurs, de 2 à 50% en poids d'éther d'allyl à chaîne grasse de formule (V), et de 0 à 1% en poids d'un agent réticulant qui est un monomère insaturé polyéthylénique copolymérisable bien connu, comme le phtalate de diallyl, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société CIBA sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).

Les polymères amphiphiles anioniques peuvent être également choisis parmi ceux comportant au moins un motif hydrophile de type acide carboxylique insaturé oléfinique, et au moins un motif hydrophobe exclusivement de type ester d'alkyl (C₁₀-C₃₀) d'acide carboxylique insaturé, utilisés selon l'invention, sont choisis de préférence parmi ceux dont le motif hydrophile de type acide carboxylique insaturé oléfinique correspond au monomère de formule (VI) suivante : 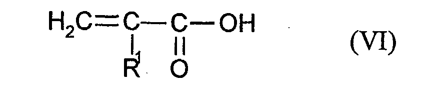 formule dans laquelle, R¹ désigne H ou CH₃ ou C₂H₅, c'est-à-dire des motifs acide acrylique, acide méthacrylique ou acide éthacrylique et dont le motif hydrophobe de type ester d'alkyl (C₁₀-C₃₀) d'acide carboxylique insaturé correspond au monomère de formule (VII) suivante :

H₂C=CR¹-CO-OR² (VII)

formule dans laquelle, R¹ désigne H ou CH₃ ou C₂H₅ (c'est-à-dire des motifs acrylates, méthacrylates ou éthacrylates) et de préférence H (motifs acrylates) ou CH₃ (motifs méthacrylates), R² désignant un radical alkyle en C₁₀-C₃₀, et de préférence en C₁₂-C₂₂.

Des esters d'alkyls (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères amphiphiles anioniques de ce type sont par exemple décrits et préparés, selon les brevets US-3 915 921 et 4 509 949.

Les polymères amphiphiles anioniques utilisables dans le cadre de la présente invention peuvent désigner plus particulièrement des polymères formés à partir d'un mélange de monomères comprenant :
(i) essentiellement de l'acide acrylique, un ester de formule (VIII) suivante

   H₂C=CR¹-CO-OR² (VIII)

   dans laquelle R¹ désigne H ou CH₃, R² désignant un radical alkyle ayant de 12 à 22 atomes de carbone, et un agent réticulant, tels que par exemple ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant,
(ii) essentiellement de l'acide acrylique et du méthacrylate de lauryle tel que celui formé à partir de 66% en poids d'acide acrylique et 34% en poids de méthacrylate de lauryle.
   Ledit réticulant est un monomère contenant un groupe 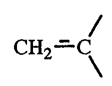 avec au moins un autre groupement polymérisable dont les liaisons insaturées sont non conjuguées l'une par rapport à l'autre. On peut notamment citer les polyallyléthers tels que notamment le polyallylsucrose et le polyallylpentaérythritol.
   Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.C. sous la dénomination COATEX SX.
   Comme polymères amphiphiles anioniques à chaînes grasses, on peut également citer le copolymère acide méthacrylique/acrylate de méthyle/diméthylméta- isopropényl benzyl isocyanate d'alcool éthoxylé commercialisé sous la dénomination VISCOPHOBE DB 1000 par la société AMERCHOL.
   Les polymères amphiphiles cationiques utilisés dans la présente invention sont choisis de préférence parmi les dérivés de cellulose quaternisée et les polyacrylates à groupements latéraux aminés. Les dérivés de cellulose quaternisée sont, en particulier,
   - les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
   - les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.
   Les polyacrylates à groupements latéraux aminés, quaternisés ou non, possèdent par exemple des groupements hydrophobes du type stéareth 20 (alcool stéarylique polyoxyéthyléné(20)) ou alkyl(C10-C30)PEG-20 itaconate.
   Les radicaux alkyle portés par les celluloses ou hydroxyéthylcelluloses quaternisées ci-dessus comportent de préférence de 8 à 30 atomes de carbone.
   Les radicaux aryle désignent de préférence les groupements phényle, benzyle, naphtyle ou anthryle.
   On peut indiquer comme exemples d'alkylhydroxyéthyl-celluloses quaternisées à chaînes grasses en C₈₋₃₀, les produits QUATRISOFT LM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C₁₈) commercialisés par la société CRODA;
   Comme exemples de polyacrylates à chaînes latérales aminées, on peut citer les polymères 8781-124B ou 9492-103 ou STRUCTURE PLUS de la société NATIONAL STARCH.
   A titre de polymères amphiphiles amphotère contenant au moins une chaîne grasse, on peut citer les copolymères de chlorure de méthacrylamidopropyl triméthylammonium/acide acrylique/méthacrylate d'alkyle en C10-C30, le radical alkyle étant de préférence un radical stéaryle.
   De préférence, les épaississants associatifs des compositions cosmétiques conformes à la présente invention présentent avantageusement en solution ou en dispersion, à 1 % de matière active dans l'eau, une viscosité mesurée au moyen du rhéomètre Rhéomat RM 180, à 25 °C, supérieure à 0,1 ps, et plus avantageusement encore supérieure à 0,2 cp, à un taux de cisaillement de 200 s⁻¹.
(ii) Parmi les homopolymères d'acide acrylique réticulés, on peut citer ceux réticulés par un éther allylique d'alcool de la série du sucre, comme par exemple les produits vendus sous les noms CARBOPOLS 980, 981, 954, 2984 et 5984 par la société GOODRICH ou les produits vendus sous les noms SYNTHALEN M et SYNTHALEN K par la société 3 VSA.
(iii) Parmi les copolymères réticulés d'acide (méth)acrylique et d'acrylate d'alkyle en C₁-C₆, on peut citer le produit vendu sous la dénomination VISCOATEX 538C par la société COATEX qui est un copolymère réticulé d'acide méthacrylique et d'acrylate d'éthyle en dispersion aqueuse à 38% de Matière Active ou le produit vendu sous la dénomination ACULYN 33 par la société ROHM & HAAS qui est un copolymère réticulé d'acide acrylique et d'acrylate d'éthyle en dispersion aqueuse à 28% de Matière Active.
(iv) Parmi les homopolymères ou copolymères non-ioniques contenant des monomères à insaturation éthylénique de type ester et/ou amide, on peut citer les produits vendus sous les dénominations de : CYANAMER P250 par la société CYTEC (polyacrylamide); PMMA MBX-8C par la société US COSMETICS (copolymère méthacrylate de méthyle /diméthacrylate d'éthylèneglycol); ACRYLOID B66 par la société RHOM & HAAS (copolymère méthacrylate de butyle / méthacrylate de méthyle); BPA 500 par la société KOBO (polyméthacrylate de méthyle).
(v) Parmi les homopolymères d'acrylate d'ammonium, on peut citer le produit vendu sous le nom MICROSAP PAS 5193 par la société HOECHST.
   Parmi les copolymères d'acrylate d'ammonium et d'acrylamide, on peut citer le produit vendu sous le nom BOZEPOL C NOUVEAU ou le produit PAS 5193 vendus par la société HOECHST (ils sont décrits et préparés dans les documents FR-2416723, US-2798053 et US-2923692).
(vi) Les polysaccharides épaississant sont notamment choisis parmi les glucanes, les amidons modifiés ou non (tels que ceux issus, par exemple, de céréales comme le blé, le maïs ou le riz, de légumes comme le pois blond, de tubercules comme les pommes de terre ou le manioc), l'amylose, l'amylopectine, le glycogène les dextranes, les celluloses et leurs dérivés (méthylcelluloses, hydroxyalkylcelluloses, éthylhydroxyéthylcelluloses, carboxyméthyl-celluloses), les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, l'acide alginique et les alginates, les arabinogalactanes, les carraghénanes, les agars, les glycosaminoglucanes, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar) et les gommes de xanthane, et leurs mélanges.

D'une manière générale, les composés de ce type, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc.", le contenu de ces trois ouvrages étant totalement inclus dans la présente demande à titre de référence.

On utilisera de préférence, les amidons, les gommes de guar, les celluloses et leurs dérivés.

Les gommes de guar peuvent être modifiées ou non modifiées.
Les gommes de guar non modifiées sont par exemple les produits vendus sous la dénomination VIDOGUM GH 175 par la société UNIPECTINE, et sous les dénominations MEYPRO-GUAR 50 et JAGUAR C par la société MEYHALL.
Les gommes de guar non-ioniques modifiées sont notamment modifiées par des groupements hydroxyalkyle en C₁-C₆.

Parmi les groupements hydroxyalkyle, on peut mentionner à titre d'exemple, les groupements hydroxyméthyle, hydroxyéthyle, hydroxypropyle et hydroxybutyle.
Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.
Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.
De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHODIA CHIMIE (MEYHALL) ou sous la dénomination GALACTASOL 4H4FD2 par la société AQUALON.

Parmi les celluloses on utilise notamment les hydroxyéthyl cellulose, les hydroxypropylcelluloses. On peut citer les produits vendus sous les dénominations KLUCEL EF, KLUCEL H, KLUCEL LHF, KLUCEL MF, KLUCEL G, par la société AQUALON.

Les alcools gras sont notamment choisis parmi l'alcool myristylique, l'alcool cétylique, l'alcool stéarylique, l'alcool béhénylique.
Les épaississants minéraux sont notamment choisis parmi les argiles.

Selon l'invention, le ou les agents épaississants peuvent représenter de 0,001 % à 20 % en poids, de préférence de 0,01 % à 10% en poids et plus particulièrement de 0,1 à 3% en poids par rapport au poids total de la composition finale.

Les compositions de l'invention contiennent en outre avantageusement au moins un agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, _-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools polyéthoxylés, polypropoxylés ou polyglycérolés, les alpha-diols polyéthoxylés, polypropoxylés ou polyglycérolés, les alkylphénols polyéthoxylés, polypropoxylés ou polyglycérolés ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle linéaire ou ramifié en C₅-C₂₀ provenant d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)₂ -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle linéaire ou ramifié, saturé ou non, en C₅-C₂₀ provenant d'un acide R₅ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL C2M concentré par la société RHODIA CHIMIE.

### (iv) Tensioactif(s) cationique(s) :

Les tensioactifs cationiques peuvent être choisis parmi :
A) les sels d'ammonium quaternaires de la formule générale (XII) suivante : 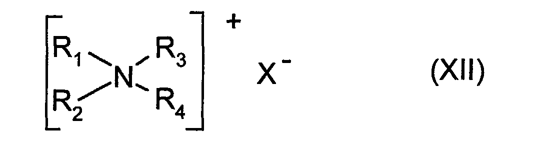
dans laquelle X- est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
, et
i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
   R4 désigne un radical alkyle, linéaire ou ramifié, comportant de 16 à 30 atomes de carbone.
   De préférence le tensioactif cationique est un sel (par exemple chlorure) de béhényl triméthyl ammonium.
ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
   R3 et R4, identiques ou différents, désignent un radical alkyle, linéaire ou ramifié, comportant de 12 à 30 atomes de carbone, ledit radical comprenant au moins une fonction ester ou amide.
   R3 et R4 sont notamment choisis parmi les radicaux alkyl(C₁₂-C₂₂)amido alkyle(C₂-C₆), alkyl(C₁₂-C₂₂)acétate ;
   De préférence le tensioactif cationique est un sel (par exemple chlorure) de stéaramidopropyl diméthyl (myristylacétate) ammonium.
B) - les sels d'ammonium quaternaire de l'imidazolinium, comme par exemple celui de formule (XIII) suivante : 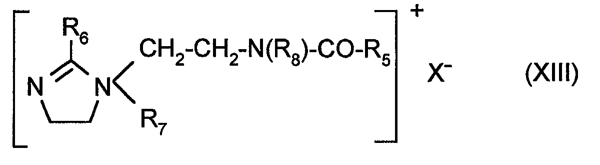 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) - les sels de diammonium quaternaire de formule (XIV) : 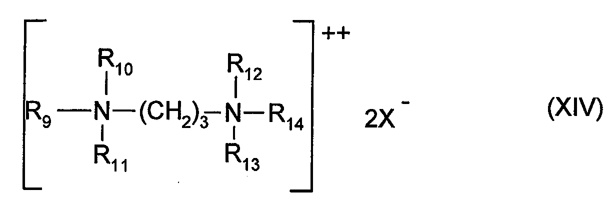 dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire contenant au moins une fonction ester de formule (XV) suivante : 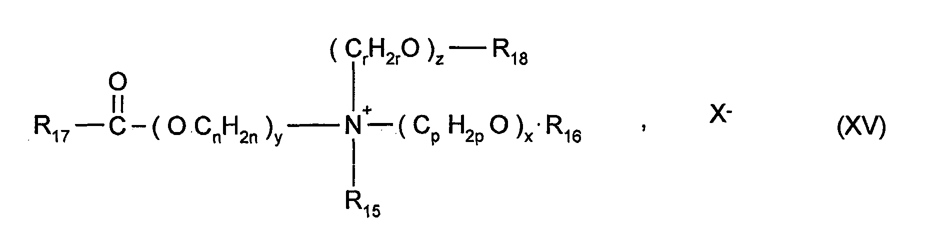
dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :

- le radical 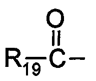
- les radicaux R₂₀ hydrocarbonés en C₁-C₂₂ linéaires ou ramifiés, saturés ou insaturés,
- l'atome d'hydrogène,
- R₁₈ est choisi parmi :

- le radical 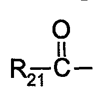
- les radicaux R₂₂ hydrocarbonés en C₁-C₆ linéaires ou ramifiés, saturés ou insaturés,
- l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (XV) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
- le radical 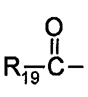
- les radicaux méthyle, éthyle ou hydrocarbonés en C₁₄-C₂₂
- l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
- le radical 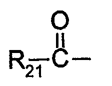
- l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

On utilise de préférence comme agent tensioactif anionique les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélanges avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;
- soit un agent tensioactif amphotère tel que les alkylbétaïnes en particulier la cocobétaïne commercialisée sous la dénomination "DEHYTON® AB 30" en solution aqueuse à 32 % de MA par la société COGNIS ou tel que les alkyl (C₈-C₂₀) amidoalkyl (C₁-C₆) bétaïnes en particulier la TEGOBETAINE® F 50 commercialisée par la société GOLDSCHMIDT.

Les composés préférés sont les alcanols en C₁-C₄ linéaires ou ramifiés, et en particulier l'éthanol et l'isopropanol.

Les polyols ont de préférence une masse moléculaire inférieure à 1000. Ils peuvent être linéaires ou ramifiés et posséder entre 2 à 10 fonctions hydroxy. Parmi ceux-ci, on peut citer le propylène glycol, le glycérol, l'héxylèneglycol, le néopentylglycol, l'isoprèneglycol, le 1,4-butanediol, le 2-méthyl-1,3-propanediol et les polyéthylèneglycols.

Les adjuvants décrits sont en général présents en quantité comprise pour chacun d'eux entre 0,01 et 20 % en poids par rapport au poids de la composition.

La composition tinctoriale conforme à l'invention peut en outre contenir des colorants directs différents de ceux de formule (I), ces colorants pouvant notamment être choisis parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs méthiniques, les colorants directs triarylméthaniques, les colorants directs indoaminiques et les colorants directs naturels.

Parmi les colorants directs benzéniques, on peut citer notamment les composés suivants:
- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, on peut citer les colorants azoiques cationiques décrits dans les demandes de brevets WO 95/15144, WO-95/01772, EP-714954 et WO 01/66646 dont le contenu fait partie intégrante de l'invention.

Parmi ces composés, on peut tout particulièrement citer les colorants suivants:
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants triarylméthaniques, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7.

Parmi les colorants indoaminiques, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La proportion totale en colorants directs additionnels est de préférence de 0,001 à 20% en poids environ du poids total de la composition et encore plus préférentiellement de 0,005 à 10% en poids environ.

La composition de l'invention peut en outre comprendre un agent oxydant. Cet agent oxydant peut être n'importe quel agent oxydant utilisé de façon classique pour la décoloration des fibres kératiniques humaines. L'agent oxydant est choisi de préférence parmi le peroxyde d'hydrogène, le peroxyde d'urée, les bromates de métaux alcalins, les persels tels que les perborates et les persulfates, les peracides et les enzymes parmi lesquelles on peut citer les peroxydases, les oxydoréductases à 2 électrons telles que les uricases et les oxygénases à 4 électrons comme les laccases. L'utilisation du peroxyde d'hydrogène est particulièrement préférée.

Lorsque la composition selon l'invention est destinée à une teinture d'oxydation classique, elle comprend en outre une base d'oxydation. Cette base d'oxydation peut être choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl para-phénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La composition selon l'invention peut contenir en outre un ou plusieurs coupleurs conventionnellement utilisés pour la teinture d'oxydation classique de fibres kératiniques humaines. Parmi ces coupleurs, on peut notamment citer les métaphénylènediamines, les méta-aminophénols, les métadiphénols, les coupleurs naphtaléniques et les coupleurs hétérocycliques.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont en général présents en quantité comprise entre 0,001 et 10 % en poids environ du poids total de la composition tinctoriale et plus préférentiellement de 0,005 à 6 %. La ou les bases d'oxydation sont présentes en quantité de préférence comprise entre 0,001 à 10 % en poids environ du poids total de la composition tinctoriale, et plus préférentiellement de 0,005 à 6 %.

D'une manière générale, les sels d'addition avec un acide utilisables dans le cadre des compositions tinctoriales de l'invention pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Le milieu cosmétiquement acceptable appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 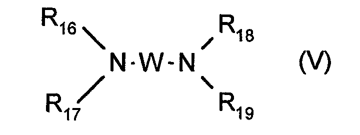 dans laquelle W est un reste propylène éventuellement substitué par un groupement hydroxyle ou un radical alkyle en C₁-C₄ ; R₁₆, R₁₇, R₁₈ et R₁₉, identiques ou différents, représentent un atome d'hydrogène, un radical alkyle en C₁-C₄ ou hydroxyalkyle en C₁-C₄.

Les compositions de l'invention peuvent aussi comprendre d'autres additifs tels que les agents anti-oxydants, les agents de pénétration, les agents séquestrants, les parfums, les agents dispersants, agents de conditionnement tels que les silicones volatiles ou non volatiles, modifiées ou non modifiées, les corps gras dont les céramides et les alcools gras, les agents conservateurs, les agents opacifiants, les polymères anioniques, cationiques

La composition tinctoriale selon l'invention peut se présenter sous des formes diverses, telles que sous forme de liquides, de crèmes, de gels, ou sous toute autre forme appropriée pour réaliser une teinture des fibres kératiniques humaines, et notamment des cheveux.

L'invention a aussi pour objet un procédé de teinture directe qui comprend l'application d'une composition tinctoriale contenant un colorant de formule (I) telle que définie précédemment sur les fibres kératiniques humaines. Après un temps de pause, les fibres sont rincées laissant apparaître des fibres colorées.

L'application sur les fibres de la composition tinctoriale contenant le colorant de formule (I) peut être mise en oeuvre en présence d'agent oxydant ce qui provoque la décoloration de la fibre (teinture directe éclaircissante). Cet agent oxydant peut être ajouté à la composition contenant le colorant direct dissymétrique polycationique au moment de l'emploi ou directement sur la fibre.

L'invention a aussi pour objet un procédé de teinture d'oxydation qui comprend l'application sur les fibres kératiniques humaines d'une composition tinctoriale qui comprend un colorant de formule (I), au moins une base d'oxydation et optionnellement au moins un coupleur, en présence d'un agent oxydant.

La base d'oxydation, le coupleur et l'agent oxydant sont tels que définis précédemment.

La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée sur les fibres simultanément ou séquentiellement à la composition tinctoriale.

Dans le cas de la teinture d'oxydation ou de la teinture directe éclaircissante, la composition tinctoriale est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres. Après un temps de pause de 3 à 50 minutes environ, de préférence 5 à 30 minutes environ, les fibres sont rincées, lavées au shampooing, rincées à nouveau puis séchées.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques humaines et tels que définis précédemment.

La composition qui est finalement appliquée sur les fibres peut se présenter sous des formes diverses, telles que sous forme de liquides, de crèmes, de gels ou sous toute autre forme appropriée pour réaliser une teinture des fibres kératiniques humaines, et notamment des cheveux.

Un autre objet de l'invention est un dispositif à plusieurs compartiments ou "kit" de teinture, et de préférence à 2 compartiments, dans lequel un premier compartiment renferme la composition tinctoriale de l'invention et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913 au nom de la demanderesse.

Les exemples suivants de compositions de décoloration et de coloration simultanée sont destinés à illustrer l'invention sans présenter un caractère limitatif.

### EXEMPLE 1:

On a préparé la composition de teinture suivante :

| | |
|---|---|
| paratoluènesulfonate d' Ammonium, [2-[p-[[2-chloro-4-(méthylsulfonyl)phényl]azo]-N-éthylanilino]éthyl][2-[N-éthyl-p-[(5-nitro-2-thiazolyl)azo]anilino]éthyl]diméthyl | 0,71 g |
| Alcool benzylique | 4,0 g |
| Polyéthylèneglycol 60E | 6,0 g |
| Hydroxyéthylcellulose | 0,7 g |
| Alkylpolyglucoside en solution aqueuse à 60% M.A* | 4,5 g M.A |
| Tampon phosphate q.s pH | 7 |
| Eau déminéralisée q.s.p | 100, g |

| | |
|---|---|
| * Matière Active | |

On a appliqué la composition ci-dessus sur des mèches de cheveux gris naturels ou permanentés à 90% de blancs et on a laissé poser pendant 20 minutes. Après rinçage à l'eau courante et séchage, les cheveux ont été teints dans une nuance vert olive

### EXEMPLE 2 :

On a préparé la composition de teinture suivante :

| | |
|---|---|
| Chlorure de (2-{[4-(2-Chloro-4-nitro-phénylazo)-2-méthoxy-5-méthyl-phényl]-méthyl-amino}-éthyl)-(2-{[4-(2-chloro-4-nitro-phénylazo)-phényl]-eéhyl-amino}-éthyl)-diméthyl-ammonium | 074 g |
| Diéthanolamide oléïque..... | 3 g |
| Acide laurique | 1 g |
| Monoéthyléther de l'éthylèneglycol | 5 g |
| Hydroxyéthylcellulose | 2 g |
| 2-amino-2-méthyl-1-propanol q.s pH | 9,5 |
| Eau déminéralisée q.s.p | 100 g |

On a appliqué la composition ci-dessus sur des mèches de cheveux gris naturels ou permanentés à 90% de blancs et on a laissé poser pendant 30 minutes. Après rinçage à l'eau courante et séchage, les cheveux ont été teints dans une nuance rouge

## Revendications

1. Composition tinctoriale pour la teinture des fibres kératiniques humaines, et en particulier des cheveux, comprenant, dans un milieu approprié pour la teinture, au moins un colorant direct polycationique dissymétrique de formule (I) :
Col₁-Z-Col₂ (I)
dans laquelle
Col₁ et Col₂ sont des colorants différents de par leur structure choisis dans le groupe formé par les colorants azoïques, les colorants méthiniques, les colorants azométhiniques et les colorants phénothiaziniques, les colorants triarylméthaniques, les colorants xanthéniques, les colorants phénanthridiniques et les colorants phtalocyanines,
Z est un groupement hydrocarboné en C₁-C₂₀, linéaire ou ramifié, saturé, insaturé ou cyclique comprenant au moins un atome d'azote et porteur d'au moins une charge cationique.

2. Composition selon la revendication 1 comprenant au moins un colorant direct polycationique dissymétrique de formule (I) dans laquelle Z, portant au moins deux charges cationiques, a pour structure la formule (II) :
-Z₁-Z₂-Z₃- (II)
dans laquelle Z₁ et Z₃, indépendamment l'un de l'autre, représente un groupe hétéroarocyclique portant un ou plusieurs hétéroatomes choisi parmi l'azote, l'oxygène, le soufre ou le phosphore ;
Z₂ est un groupe hydrocarboné linéaire ou ramifié comprenant de 0 à 10, de préférence de 2 à 6 atomes de carbone.

3. Composition selon la revendication 2 **caractérisée en ce que** les hétérocycles comprennent de 5 à 8 chaînons.

4. Composition selon la revendication 2, **caractérisée en ce qu'**au moins un des radicaux Z₁ ou Z₂ est un hétérocycle condensé avec un noyau benzénique.

5. Composition selon les revendications 2 à 4 telle que le groupe hétéroarocyclique est choisi dans le groupe formé par les pyrroles, imidazoles, isoimidazoles, pyrazoles, pyridines.

6. Composition selon la revendication 1 comprenant au moins un colorant direct polycationique dissymétrique de formule (I) dans laquelle Z a pour structure la formule (III) 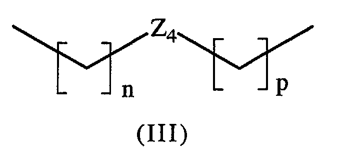 dans laquelle
n représente un nombre entier de 1 à 10, de préférence de 2 à 5,
p représente un nombre entier de 1 à 10, de préférence de 2 à 5,
Z₄ représente un groupe cationique et de préférence dicationique comprenant entre 2 et 16 atomes de carbone, de préférence entre 5 et 12 atomes de carbone, ce groupe étant aliphatique saturé ou insaturé, carbocyclique ou polycarbocyclique, aromatique ou polyaromatique, hétéroaromatique ou polyhétéroaromatique, substitué ou non substitué par un à cinq substituants choisis parmi les groupements hydroxy, carboxy, alcoxy en C₁-C₄carbonyles, hydrogénocarbonyles, alcoxy en C₁-C₄, acyle en C₂-C₄, amino, mono ou dialkylamino, mono ou di(hydroxyalkyl en C₁-C₄)amino, cyano, nitro, sulfonato.

7. Composition selon la revendication 1 telle qu'elle comprend au moins un colorant direct polycationique dissymétrique de formule (I) dans laquelle Z est un groupement aliphatique.

8. Composition selon la revendication 7, **caractérisée en ce qu'**elle comprend au moins un colorant direct polycationique dissymétrique de formule (I) dans laquelle Z est un groupe aliphatique de formule (IV) : 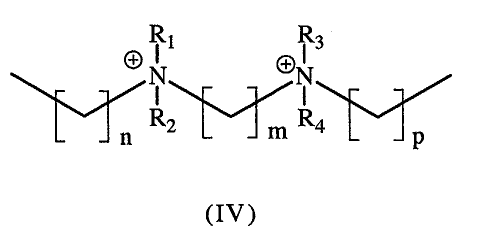 où n représente un nombre entier de 1 à 10, de préférence de 2 à 5,
m représente un nombre entier de 1 à 15, de préférence de 2 à 10,
p représente un nombre entier de 1 à 10, de préférence de 2 à 5,
R₁, R₂, R₃ et R₄ désignent indépendamment les uns des autres un radical alkyle en C₁-C₆.

9. Composition selon l'une quelconques des revendications 1 à 8, **caractérisée en ce que** les colorants Col₁ et Col₂ ont le même nombre de charges cationiques.

10. Composition selon la revendication 9, **caractérisée en ce que** Col₁ et Col₂ portent chacun une charge cationique.

11. Composition selon l'une quelconque des revendications précédentes, **caractérisée par le fait que** le direct polycationique dissymétrique de formule (I) est choisi dans le groupe formé par les :
Ammonium, [2-[p-[[2-chloro-4-(méthylsulfonyl)phényl]azo]-N-éthylanilino]éthyl][2-[N-éthyl-p-[(5-nitro-2-thiazolyl)azo]anilino]éthyl]diméthyl-, p-toluènesulfonate
(2-{[4-(2-Chloro-4-nitro-phénylazo)-2-methoxy-5-méthyl-phényl]-méthyl-amino}-éthyl)-(2-{[4-(2-chloro-4-nitro-phénylazo)-phényl]-éthyl-amino}-éthyl)-diméthyl-ammonium
Tétraiodure de Quinolinium, 1-[3-[[3-[diméthyl[3-[2-[(3-méthylbenzothiazolium-2-yl)méthylène]-3(2H)-benzothiazolyl]propyl]ammonio]propyl]diméthylammonio]propyl ]-4-[(3-méthyl-2(3H)-benzothiazolylidène)méthyl]-
Tétraiodure de Quinolinium, 1-[3-[[3-[diméthyl[3-[4-[(3-méthyl-2(3H)-benzothiazolylidène)méthyl]quinolinio]propyl]ammonio]propyl]d iméthylammonio]propyl]-4-[5-(3-méthyl-2(3H)-benzothiazolylidène)-1,3-pentadiényl]-
Tétraiodure de Quinolinium, 1-[3-[[3-[diméthyl[3-[2-[5-(3-méthyl-2(3H)- benzothiazolylidène)-1,3-pentadiényl]benzothiazolium-3-yl]propyl]ammonio]propyl]diméthylammonio]propyl]-4-[(3-méthyl-2(3H)- benzothiazolylidène)méthyl]-,
Tétraiodure de Quinolinium, 1-[5-[diméthyl[3-[diméthyl[3-[4-[(3-méthyl-2(3H)-benzothiazolylidène)méthyl]quinolinio]propyl]ammonio]propyl]a mmonio]pentyl]-4-[3-(3-méthyl-2(3H)-benzothiazolylidène)-1-propenyl]-
Tétraiodure de Quinolinium, 1-[3-[[3-[[4-[2-[5-(1,3-dihydro-1,3,3-triméthyl-2H-indol-2-ylidène)-1,3-pentadiényl]benzothiazolium-3-yl]butyl]diméthylammonio]propyl]diméthylammonio]propyl]-4-[(3-méthyl-2(3H)-thiazolylidène)méthyl]-
Tétraiodure de Quinolinium, 1-[3-[[3-[[3-[2-[5-(1,3-dihydro-1,3,3-triméthyl-2H-indol-2-ylidène)-1,3-pentadiényl]benzothiazolium-3-yl]propyl]diméthylammonio]propyl]diméthylammonio]propyl]-4-[(3-méthyl-2(3H)-thiazolylidène)méthyl]-
Tétrachlorure de Quinolinium, 1-[3-[[3-[diméthyl[3-[4-[(3-méthyl-2(3H)-benzothiazolylidène)méthyl]quinolinio]propyl]ammonio]propyl]d iméthylammonio]propyl]-4-[5-(3-méthyl-2(3H)-benzothiazolylidène)-1,3-pentadiényl]-,
Tétrachlorure de Quinolinium, 1-[3-[[3-[diméthyl[3-[2-[5-(3-méthyl2(3H)- benzothiazolylidène)-1,3-pentadiényl]benzothiazolium-3-yl]propyl]ammonio]propyl]diméthylammonio]propyl]-4-[(3-méthyl-2(3H)- benzothiazolylidène)méthyl]-,
Tétrachlorure de Quinolinium, 1-[4-[[3-[diméthyl[3-[4-[(3-méthyl-2(3H)-benzothiazolylidène)méthyl]quinolinio]propyl]ammonio]propyl]d iméthylammonio]butyl]-4-[3-(3-méthyl-2(3H)-benzothiazolylidène)-1-propenyl]-,
Tétraiodure de Quinolinium, 1-[3-[[3-[[5-[2-[5-(1,3-dihydro-1,3,3-triméthyl-2H-indol-2-ylidène)-1,3-pentadiényl]benzothiazolium-3-yl]pentyl]diméthylammonio]propyl]diméthylammonio]propyl]-4-[(3-méthyl-2(3H)-benzothiazolylidène)méthyl]-,
Tétrachlorure de Quinolinium, 1-[3-[[3-[[4-[2-[5-(1,3-dihydro-1,3,3-triméthyl-2H-indol-2-ylidène)-1,3-pentadiényl]benzothiazolium-3-yl]butyl]diméthylammonio]propyl]diméthylammonio]propyl]-4-[(3-méthyl-2(3H)-benzothiazolylidène)méthyl]-,
Tétrachlorure de Quinolinium, 1-[3-[[3-[[3-[2-[5-(1,3-dihydro-1,3,3-triméthyl-2H-indol-2-ylidène)-1,3-pentadiényl]benzothiazolium-3-yl]propyl]diméthylammonio]propyl]diméthylammonio]propyl]-4-[(3-méthyl-2(3H)-benzothiazolylidène)méthyl]-
Tétrachlorure de Quinolinium, 1-[6-[[3-[diméthyl[3-[4-[(3-méthyl-2(3H)-benzothiazolylidène)méthyl]quinolinio]propyl]ammonio]propyl]d iméthylammonio]hexyl]-4-[3-(3-méthyl-2(3H)-benzothiazolylidène)-1-propenyl]-,
Tétrachlorure de Quinolinium, 1-[5-[[3-[diméthyl[3-[4-[(3-méthyl-2(3H)-benzothiazolylidène)méthyl]quinolinio]propyl]ammonio]propyl]d iméthylammonio]pentyl]-4-[3-(3-méthyl-2(3H)-benzothiazolylidène)-1-propenyl]-,
Tétraiodure de Naphtho[1,2-d]thiazolium, 2,2'-[1,3-propanediylbis[(diméthyliminio)-3,1-propanediyl-1(4H)-quinolinyl-4-ylidèneméthylidyne]]bis[1-méthyl-,
Tétrachlorure de Phénanthridinium, 3,8-diamino-5-[3-[[3-[diméthyl[3-[4-[(3-méthyl-2(3H)-benzothiazolylidène)méthyl]quinolinio]propyl]ammonio]propyl]d iméthylammonio]propyl]-6-phényl]-
Quinolinium, 1-[3-[diméthyl[3-[diméthyl[3-[4-[(3-méthyl-2(3H)-benzothiazolylidène)méthyl]quinolinio]propyl]iminio]propyl]imi nio]propyl]- 4-[3-(3-méthyl-2(3H)-benzothiazolylidène)-1-propenyl]-

12. Composition selon l'une quelconque des revendications précédentes, **caractérisée par le fait que** le ou les colorants directs polycationiques dissymétriques de formule (I) sont présents à une concentration variant de 0,001 à 5% en poids par rapport au poids total de la composition tinctoriale, et de préférence de 0,05 à 2%.

13. Composition selon l'une quelconque des revndications précédentes, **caractérisée par le fait qu'**elle contient un adjuvant cosmétique choisi parmi les monoalcools, les polyols, les agents tensio-actifs anioniques, cationiques, non ioniques, amphotères ou zwittérioniques, ou leurs mélanges, les agents épaississants minéraux ou organiques, et en particulier les polymères associatifs anioniques, cationiques, non ioniques et amphotères.

14. Composition selon l'une quelconque des revendications précédentes, **caractérisée par le fait que** les adjuvants sont présents en quantité comprise pour chacun d'eux entre 0,01 et 20 % en poids par rapport au poids total de la composition tinctoriale.

15. Composition selon l'une quelconque des revendications précédentes comprenant en outre au moins un colorant direct différent de ceux de formule (I), choisi parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs méthiniques, les colorants directs triarylméthaniques, les colorants directs indoaminiques et les colorants directs naturels.

16. Composition selon l'une quelconque des revendications précédentes comprenant en outre un agent oxydant, de préférence le peroxyde d'hydrogène.

17. Composition selon l'une quelconque des revendications précédentes comprenant de plus une base d'oxydation.

18. Composition selon la revendication 17 dans laquelle la base d'oxydation est choisie parmi les paraphénylènediamines, les bis-phénylalkylènediamines, les para-aminophénols, les ortho-aminophénols, les bases hétérocycliques, et leurs sels d'addition avec un acide.

19. Composition selon l'une quelconque des revendications 16 ou 17 comprenant en outre au moins un coupleur.

20. Composition selon la revendication 19 dans laquelle le coupleur est choisi parmi les métaphénylènediamines, les méta-aminophénols, les métadiphénols, les coupleurs naphtaléniques et les coupleurs hétérocycliques et leur sel d'addition avec un acide.

21. Procédé de teinture directe des fibres kératiniques humaines et plus particulièrement des cheveux, **caractérisé en ce qu'**on applique sur les fibres au moins une composition tinctoriale telle que définie à l'une quelconque des revendications 1 à 15.

22. Procédé selon la revendication 21 mettant en oeuvre un agent oxydant.

23. Procédé selon la revendication 22 dans lequel l'agent oxydant est mélangé au moment de l'emploi à la composition tinctoriale.

24. Procédé selon la revendication 22 dans lequel l'agent oxydant est appliqué sur les fibres sous forme de composition oxydante simultanément ou séquentiellement à la composition tinctoriale.

25. Procédé de teinture d'oxydation des fibres kératiniques humaines et plus particulièrement des cheveux, **caractérisé en ce qu'**on applique sur les fibres au moins une composition tinctoriale telle que définie à l'une quelconque des revendications 1 à 15, comprenant de plus au moins une base d'oxydation et optionnellement au moins un coupleur, en présence d'un agent oxydant.

26. Procédé selon la revendication 25 dans lequel l'agent oxydant est mélangé au moment de l'emploi à la composition tinctoriale.

27. Procédé selon la revendication 25 dans lequel l'agent oxydant est appliqué sur les fibres sous forme de composition oxydante simultanément ou séquentiellement à la composition tinctoriale.

28. Dispositif à deux compartiments, ou "kit", pour la teinture des fibres kératiniques humaines, et plus particulièrement des cheveux , dans lequel un premier compartiment contient une composition telle que définie à l'une quelconque des revendications 1 à 15 et 17 à 20 et un deuxième compartiment contient une composition oxydante.

29. Utilisation des composés directs polycationiques dissymétrique de formule (I) telle que définie selon l'une quelconque des revendications 1 à 11, à titre de colorants directs, dans une, ou pour la préparation d'une composition de teinture des fibres kératiniques humaines et plus particulièrement des cheveux.
